# FASCICULE DE BREVET EUROPEEN

(11) **EP 2 567 688 B1**
(45) Date de publication et mention de la délivrance du brevet: **19.08.2015**
(21) Numéro de dépôt: 12190685.3
(22) Date de dépôt: 23.09.2008
(51) Int. Cl.: A61K 8/64, A61Q 5/00, A61Q 19/00, A61Q 19/08, A61K 8/99, A61Q 7/00

(54) **UTILISATION D'UN AGENT NATUREL DANS DES COMPOSITIONS COSMETIQUES**
Verwendung einer natürlichen Substanz in kosmetischen Zusammensetzungen
Use of a natural agent in cosmetic compositions

(30) Priorité: 25.09.2007 FR 0706701
(43) Date de publication de la demande: 13.03.2013
(62) Demande divisionnaire de: 08860035.8
(73) Titulaire: Lesaffre et Compagnie, 75001 Paris (FR)
(72) Inventeur: Borreill, Dominique, Marie, Noëlle, 94430 CHENNEVIERES SUR MARNE (FR); Jüsten, Peter, 92500 RUEIL MALMAISON (FR)
(74) Mandataire: Cabinet Plasseraud

(56) Documents cités:
- J.A.T. BARRIGA, D.G. COOPER, E.S. IDZIAK, D.R. CAMERON: "Components of the bioemulsifier from S. cerevisiae", ENZYME MICROB. TECHNOL., vol. 25, 1999, pages 96-102, XP002690474,
- TORABIZADEH H ET AL: "Preparation and characterisation of bioemulsifier from Saccharomyces cerevisiae and its application in food products", LWT- FOOD SCIENCE AND TECHNOLOGY, ACADEMIC PRESS, UNITED KINGDOM, vol. 29, no. 8, 1 janvier 1996 (1996-01-01), pages 734-737, XP000929219, ISSN: 0023-6438, DOI: 10.1006/FSTL.1996.0114
- CAMERON ET AL: "The Mannoprotein of Saccharomyces cerevisiae is an effective Bioemulsifier", APPLIED AND ENVIRONMENTAL MICROBIOLOGY, AMERICAN SOCIETY FOR MICROBIOLOGY, US, vol. 54, no. 6, 1 juin 1988 (1988-06-01), pages 1420-1425, XP002087061, ISSN: 0099-2240

## Description

### DOMAINE DE L'INVENTION

La présente invention a pour objet l'utilisation d'un nouvel agent naturel dans des compositions cosmétiques, ainsi que dans des compositions dermatologiques.

### ARRIERE-PLAN TECHNOLOGIQUE

Les mannoprotéines sont des mannanes liés à des protéines que l'on trouve notamment dans la paroi des levures où elles ont une fonction structurale et /ou antigénique.

Les mannoprotéines sont couramment utilisées en oenologie, notamment pour la stabilisation du vin (voir par exemple les documents WO 96/013571, WO 01/046380 et WO 06/067147). L'utilisation de mannoprotéines pour réduire la turbidité de boissons alcoolisées est décrite dans la demande WO 07/034986.

Dans la demande de brevet EP 0 790316, les mannoprotéines sont utilisées comme agent émulsifiant dans des boissons et produits alimentaires.

Dans la demande de brevet WO 01/066574, les mannoprotéines de *cryptococcus neoformans* sont utilisées pour la préparation d'un médicament destiné au traitement de maladies inflammatoires ou d'infections, via un mode d'administration par voie systémique. Les mannoprotéines permettent alors de freiner la migration des neutrophiles.

Par ailleurs, les mannoprotéines sont décrites comme adjuvant de substances actives, c'est-à-dire comme un composé permettant d'améliorer ou de renforcer l'effet thérapeutique de substances actives.

Ainsi, dans la demande internationale US 2005/0226822, les mannoprotéines sont utilisées comme adjuvant de l'ovomucine dans des compositions de soin buccal.

La demande internationale WO 06/036817 décrit l'utilisation de mannoprotéines comme adjuvant dans une composition pharmaceutique comprenant des cellules ou des extraits de cellules de champignon génétiquement modifié pour avoir au moins une propriété filamenteuse altérée. Les propriétés immunogènes des mannoprotéines sont utilisées pour améliorer la réponse immune de la composition. Cette demande repose sur un principe de vaccination contre des infections fongiques dues notamment à des champignons du genre candida.

Dans la demande de brevet US 2005/0191268, les mannoprotéines sont utilisées comme adjuvant des polyphénols dans des préparations cosmétiques comprenant un extrait d'un résidu du procédé de fabrication du vin et un ou plusieurs additifs ou auxiliaires cosmétiques. Ledit résidu est le dépôt restant au fond des cuves après avoir pompé le vin en fin de fermentation. Ledit extrait de résidu comprend un ou plusieurs polyphénols et une ou plusieurs protéines, par exemple des mannoprotéines, en tant que complexe d'association.

Le document J.A.T. BARRIGA et al. (Enzyme Microbiol Technol, vol. 25, 1999, p. 96-102) décrit l'utilisation de mannoprotéines issues de *Saccharomyces cerevisiae* comme bioémulsifiant pour la fabrication d'émulsions, dans le domaine cosmétique et alimentaire.

Dans le domaine cosmétique, il est utile de disposer d'agents multifonctionnels qui permettent en particulier de diminuer la palette de matières premières nécessaires à la préparation de différentes compositions cosmétiques et/ou de simplifier le procédé de préparation des produits cosmétiques.

De plus, les consommateurs sont à la recherche de produits cosmétiques naturels, comprenant des composants d'origine naturelle. Il est donc utile de disposer d'agents fonctionnels naturels pour la préparation de produits cosmétiques.

Il est décrit dans la présente invention un nouvel agent naturel multifonctionnel utile dans le domaine cosmétique ou dermatologique.

Il est également décrit une méthode de traitement cosmétique ou dermatologique de la peau et/ou des phanères.

Il est décrit dans la présente invention une composition cosmétique ou dermatologique comprenant des mannoprotéines à titre de substance active.

En effet, les mannoprotéines sont en fait des principes actifs en tant que telles, il n'est donc pas nécessaire de les associer avec d'autres composants, notamment pour une application topique dans des compositions cosmétiques et/ou dermatologiques.

### RESUME DE L'INVENTION

Un objet de l'invention est de fournir de nouvelles compositions cosmétiques ou dermatologiques.

La présente invention a pour objet une composition cosmétique ou dermatologique comprenant des mannoprotéines à titre d'additif

L'invention repose sur la découverte que les mannoprotéines ont des effets texturants dans ces compositions cosmétiques et/ou dermatologiques qu'il est possible d'utiliser de façon avantageuse.

### DESCRIPTION DETAILEE DE MODES DE REALISATION

Selon un premier objet, la présente invention porte sur une utilisation, dans des compositions cosmétiques et/ou dermatologiques, de mannoprotéines comme agent texturant, ladite utilisation étant telle que définie à l'une quelconque des revendications 1 à 5.

Selon un autre objet, l'invention porte sur une composition cosmétique et/ou dermatologique comprenant des mannoprotéines à titre d'agent texturant, ladite composition étant telle que définie à l'une quelconque des revendications 6 à 8.

La présente invention a notamment pour objet une composition cosmétique ou dermatologique comprenant des mannoprotéines à titre d'additif, caractérisée en ce qu'elle ne contient pas de polyphénols.

La présente invention a notamment pour objet une composition telle que définie ci-dessus caractérisée en ce que les mannoprotéines sont issues de levures, notamment de levure du genre *Saccharomyces*, *Kluyveromyces*, *Torula* ou *Candida*, de préférence *Saccharomyces.*

La présente invention a particulièrement pour objet une composition telle que définie ci-dessus caractérisée en ce que les mannoprotéines sont issues de *Saccharomyces cerevisiae*.

Par « composition cosmétique », on désigne ici une composition destinée à procurer un effet cosmétique dans le cadre d'une application topique.

Par « composition dermatologique », on désigne une composition destinée à procurer un effet thérapeutique topique.

Dans les deux cas, la composition comprend en plus de l'eau et des mannoprotéines l'ensemble des composés (notamment excipients et additifs) nécessaires pour un usage cosmétique ou dermatologique, ainsi que l'homme du métier l'appréciera.

Le terme « topique » indique que la composition est active à l'endroit où elle est appliquée, sur la peau, les phanères ou des muqueuses. La composition selon l'invention peut à la fois cibler les couches superficielles de l'épiderme, l'épiderme, et / ou le derme.

La présente invention a notamment pour objet une composition cosmétique ou dermatologique telle que définie ci-dessus destinée à une application sur la peau et/ou sur les phanères, à l'exclusion des muqueuses.

Par le terme « phanère », on désigne de manière générale tout ce qui recouvre le corps, et notamment les cheveux, ongles, poils, cils.

Le terme « peau » englobe le cuir chevelu.

Les mannoprotéines selon l'invention sont obtenues par des procédés classiques bien connus de l'homme de l'art.

En particulier, les mannoprotéines sont extraites de la paroi de cellules de champignons, notamment de la paroi de cellules de levure.

L'extraction des mannoprotéines de la paroi des cellules peut être effectuée par solubilisation sélective des mannoprotéines, via une hydrolyse par voie chimique, enzymatique ou physico-chimique. De préférence, la solubilisation sélective est obtenue sous l'action de la température.

La fraction insoluble est éliminée et la fraction solubilisée contenant les mannoprotéines est récupérée. Les mannoprotéines de la fraction soluble peuvent alors être précipitées ou concentrées et éventuellement séchées.

De préférence, une étape de purification des mannoprotéines est effectuée après séparation de la fraction insoluble.

Une méthode d'obtention de mannoprotéines solubles est par exemple décrite dans la demande de brevet EP 1 094117 au nom de la Demanderesse, ou encore WO-A-96/13571 et WO-A-97/49794.

On peut utiliser tout type de produit de départ, et en particulier des souches de levures. Notamment, les mannoprotéines sont issues de *Saccharomyces*, *Kluyveromyces*, *Torula* ou *Candida.* On préfèrera *Saccharomyces*, notamment *cerevisiae.*

La présente invention a particulièrement pour objet une composition cosmétique ou dermatologique telle que définie ci-dessus, dans laquelle les mannoprotéines sont extraites de la paroi de levures, lesdites levures n'ayant servi à aucun usage avant ladite extraction des mannoprotéines.

Les compositions cosmétiques ou dermatologiques selon l'invention peuvent être préparées à partir de mannoprotéines sous forme sèche, notamment sous forme de poudre, ou en solution, par exemple en solution aqueuse.

Une composition cosmétique ou dermatologique selon l'invention comprend au moins un composé à titre de substance active, au moins un composé à titre d'additif et au moins un composé à titre d'excipient, un même composé pouvant être utilisé à plusieurs titres.

Par « substance active » ou « principe actif » ou « matière active », on désigne ici la substance responsable de l'effet cosmétique dans le cas d'une composition cosmétique ou responsable de l'effet thérapeutique dans le cadre d'une composition dermatologique.

Par « additif », on désigne un agent ayant dans la composition cosmétique ou dermatologique un rôle de conservateur, chélateur, colorant, filtre UV (permettant de protéger les matières premières), régulateur de pH (acide ou base), texturant, parfum et/ou antioxydant.

Par « excipient », on désigne des composés hydrophiles constituant une phase aqueuse, des composés hydrophobes constituant une phase grasse ou des tensioactifs.

Les composés hydrophiles de la phase aqueuse sont notamment choisis parmi l'eau, alcools et polyols.

Les composés hydrophobes de la phase grasse sont notamment choisis parmi les hydrocarbures, acides gras, alcools gras, esters, glycérides, cérides, phosphatides et silicones.

Les tensioactifs sont des molécules amphiphiles capables de maintenir ensemble deux milieux normalement non miscibles entre eux en diminuant les tensions interfaciales. Les tensioactifs sont ioniques (anioniques, cationiques ou amphotères) ou non-ioniques.

Les mannoprotéines constituent donc un nouvel agent naturel multifonctionnel particulièrement utile pour la préparation de compositions cosmétiques ou dermatologiques.

La composition cosmétique ou dermatologique selon l'invention ne contient pas de polyphénol. En particulier, la composition selon l'invention ne contient pas de dihydroxybenzène, pyrogallol, phloroglucinol, anthocyanidine, proanthocyanidine, flavone, catéchine, ni de tanin.

La présente invention a également pour objet une composition cosmétique ou dermatologique telle que définie ci-dessus, caractérisée en ce qu'elle ne contient pas d'ovomucine.

La composition cosmétique ou dermatologique selon l'invention peut comprendre, en plus des mannoprotéines telles que définies ci-dessus, d'autres constituants de levure.

La présente invention a également pour objet une composition cosmétique ou dermatologique telle que définie ci-dessus, caractérisée en ce qu'elle ne contient pas de champignon filamenteux ayant une propriété filamenteuse altérée, notamment résultant d'une altération du gène codant la protéine CRV1, ni un extrait cellulaire desdits champignons filamenteux.

Dans un autre mode de réalisation préféré, la présente invention a pour objet une composition cosmétique ou dermatologique telle que définie ci-dessus, caractérisée en ce qu'elle ne comprend aucun composant extrait de cellules fongiques autre que des mannoprotéines.

La présente invention a particulièrement pour objet une composition cosmétique ou dermatologique telle que définie ci-dessus, dans laquelle ledit additif a un effet texturant.

Par « agent texturant », on désigne un agent capable d'augmenter la viscosité des phases aqueuses dans lesquelles il est dispersé, l'augmentation étant avantageusement élevée.

Un agent texturant peut, selon les cas, être un épaississant et/ou un gélifiant.

Par « épaississant », on désigne une substance qui permet d'obtenir une solution visqueuse sans formation d'un réseau tridimensionnel, par opposition notamment aux gélifiants.

L'invention a encore pour objet l'utilisation des mannoprotéines comme agent texturant, dans des compositions cosmétiques et / ou dermatologiques.

L'invention a encore pour objet des compositions telles que décrites ci-dessus, dans lesquelles les mannoprotéines sont présentes en combinaison avec un autre produit texturant, la gomme de xanthane. Les mannoprotéines sont susceptibles d'augmenter de façon synergique l'effet texturant (en particulier la capacité à accroître la viscosité) d'autres produits texturants, et notamment de la gomme de xanthane. Un rapport massique des mannoprotéines par rapport à la gomme de xanthane compris entre 1:5 et 1:3 fournit le meilleur effet synergique. De manière plus particulièrement préférée, ce rapport massique est d'environ 1:4.

Il est également décrit dans l'invention des méthodes de traitement cosmétique utilisant ces compositions.

La composition cosmétique ou dermatologique selon l'invention peut contenir plusieurs substances actives, additifs et/ou excipients.

Il est décrit dans la présente invention une composition cosmétique ou dermatologique telle que définie ci-dessus, dans laquelle les mannoprotéines sont utilisées à titre de substance active et d'additif.

Ainsi, les mannoprotéines sont à la fois responsables de l'effet cosmétique ou dermatologique et de l'effet texturant.

La présente invention a notamment pour objet une composition cosmétique ou dermatologique telle que définie ci-dessus, dans laquelle les mannoprotéines sont également présentes à titre de tensioactif, notamment à titre d'émulsifiant.

Par « émulsifiant » ou « émulsionnant », on désigne un tensioactif qui entre dans la composition d'une émulsion, ladite émulsion comprenant également une phase lipophile et une phase hydrophile. Les émulsions sont des dispersions d'un liquide dans un autre liquide, les deux liquides étant non miscibles entre eux.

Il est décrit dans la présente invention une composition cosmétique ou dermatologique telle que définie ci-dessus comprenant des mannoprotéines à titre de substance active et à titre d'excipient.

La présente invention a pour objet une composition cosmétique ou dermatologique telle que définie ci-dessus comprenant des mannoprotéines à titre d'additif et d'excipient.

Il est également décrit dans la présente invention une composition cosmétique ou dermatologique telle que définie ci-dessus comprenant des mannoprotéines à titre de substance active, d'additif et d'excipient.

Cependant, les mannoprotéines n'ont pas forcément d'effet excipient dans la composition cosmétique ou dermatologique. En particulier, il est décrit dans la présente invention une composition cosmétique ou dermatologique telle que définie ci-dessus dans laquelle les mannoprotéines n'ont aucun effet tensioactif, et notamment aucun effet émulsifiant.

La présente invention a pour objet une composition cosmétique ou dermatologique telle que définie ci-dessus sous forme de solution (une phase), dispersion (notamment une émulsion, suspension, mousse ou aérosol), gel, huile, stick, poudre, lingettes ou patchs.

Par « émulsion », on désigne tout type d'émulsion, et notamment macro-émulsion, micro-émulsion, nano-émulsion, émulsion simple, émulsion multiple.

Les émulsions sont des dispersions d'un liquide dans un autre liquide, les deux liquides étant non miscibles. Les émulsions comportent une phase lipophile, hydrophile et un émulsionnant.

Parmi les émulsions, on trouve notamment des laits, lotions, crèmes, etc..

Les nano-émulsions sont des dispersions dont la taille des particules dispersées est inférieure à 1000 nm de diamètre, notamment de 10 nm à 100 nm.

Les micro-émulsions sont des dispersions dont la taille des particules dispersées est inférieure à 1000 µm de diamètre, notamment de 10 µm à 100 µm.

Les nano-émulsions et les micro-émulsions constituent des milieux transparents.

La composition cosmétique ou dermatologique telle que définie ci-dessus est en particulier appropriée pour des applications cutanées ou capillaires.

La composition pour des applications capillaires est notamment sous la forme de shampoings, lotions, masques, sprays.

La présente invention a pour objet une composition telle que définie ci-dessus, comprenant de 0,01 % à 20 % de mannoprotéines, notamment de 0,01 % à 15 % de mannoprotéines, notamment de 0,01 % à 10 % de mannoprotéines, notamment de 0,01 % à 3 % de mannoprotéines.

Les pourcentages sont donnés en poids/poids.

Il est notamment décrit dans la présente invention une composition telle que définie ci-dessus, comprenant de 0,01 % à 3 % de mannoprotéines, notamment de 0,01 % à 2 % de mannoprotéines, à titre de substance active.

La présente invention a notamment pour objet une composition telle que définie ci-dessus, comprenant de 0,01 % à 20 % de mannoprotéines, notamment de 1 à 20 % de mannoprotéines, notamment de 10 % à 20 % de mannoprotéines, notamment de 15 % à 20 % de mannoprotéines, à titre d'additif.

Il est également décrit dans la présente invention un procédé de préparation d'une composition cosmétique ou dermatologique telle que définie ci-dessus comprenant une étape de mélange des mannoprotéines avec un véhicule cosmétique ou dermatologique acceptable.

Le véhicule cosmétique ou dermatologique acceptable est en particulier choisi parmi les additifs et/ou excipients mentionnés ci-dessus.

Il est également décrit dans la présente un procédé de préparation d'une composition cosmétique ou dermatologique telle que définie ci-dessus comprenant une étape de mélange des mannoprotéines avec un véhicule cosmétique ou dermatologique acceptable et avec au moins une substance active autre que des mannoprotéines.

Il est également décrit dans la présente invention une méthode de traitement cosmétique comprenant une étape de mise en contact sur la peau et / ou sur les phanères d'une composition cosmétique telle que définie ci-dessus ou telle qu'obtenue par le procédé de préparation défini ci-dessus.

Le terme « mise en contact » est également appelé « application » par la suite.

La méthode de traitement peut comprendre une à plusieurs applications par jour, de préférence une à trois applications par jour.

La fréquence des applications de la composition cosmétique peut diminuer au cours du traitement.

La méthode de traitement cosmétique peut consister en un traitement court, de une à plusieurs semaines, ou un traitement à long terme sur plusieurs années. La méthode de traitement peut également consister en un traitement sous forme de cures renouvelées tous les ans ou plusieurs fois par an.

Il est également décrit dans la présente invention une méthode de traitement cosmétique telle que définie ci-dessus, destinée à hydrater l'épiderme, affermir le derme, lutter contre le vieillissement de la peau, réguler la sécrétion de sébum, réparer les cheveux et / ou améliorer la croissance des cheveux.

L'hydratation de l'épiderme vise à la fois à rétablir la qualité de la barrière cutanée, à savoir une imperméabilité limitant l'évaporation de l'eau, et à favoriser la présence de molécules piégeant l'eau, notamment les glycosaminoglycanes, dont l'acide hyaluronique.

Ladite méthode de traitement cosmétique est particulièrement utile dans le traitement et/ou la prévention de la sécheresse cutanée et des pellicules.

L'affermissement du derme vise à maintenir ou renforcer la fermeté du derme, en particulier en activant la synthèse d'élastine, la synthèse et la maturation du collagène et la contraction du lattis de collagène.

La lutte contre le vieillissement de la peau a pour objet de retarder et/ou diminuer les signes de vieillissement.

Il est également possible de coupler le traitement destiné à lutter contre le vieillissement à une hydratation de l'épiderme.

La méthode de traitement cosmétique décrite est particulièrement préconisée chez des sujets à partir de 20 ans, notamment à partir de 30 ans, notamment à partir de 40 ans, notamment à partir de 50 ans.

La régulation de la sécrétion de sébum a notamment pour objet de diminuer la sécrétion de sébum.

La méthode de traitement cosmétique est particulièrement utile pour réguler la séborrhée des peaux grasses, notamment pour des peaux grasses dites « à problèmes » ou « à tendance acnéique », et/ou pour des cheveux dits « gras ».

La réparation des cheveux consiste à reconstruire le cheveu, notamment en lissant la cuticule du cheveu et /ou à redonner de la brillance et /ou de la douceur aux cheveux.

La méthode de traitement cosmétique est notamment appropriée chez des sujets dont les cheveux sont abîmés, notamment suite à une exposition au soleil, la mer, à des lavages trop fréquents, des colorations, des balayages, des permanentes, etc.

L'amélioration de la croissance des cheveux vise à augmenter la cinétique de croissance des cheveux, appelée également pousse des cheveux.

La méthode de traitement cosmétique est notamment appropriée chez des sujets dont la cinétique de croissance des cheveux est lente et/ou en cas de perte de cheveux normale.

La perte de cheveux dite normale correspond notamment à l'alopécie androgénogénétique, l'alopécie endocrinienne, ou l'alopécie liée à l'âge.

Dans un mode de réalisation avantageux, la méthode de traitement cosmétique selon l'invention est appropriée pour une application sur le visage, en particulier sur le contour des yeux, le nez, le front, le menton, sur le corps, en particulier sur les mains, les pieds, et le dos, sur les cheveux et/ou le cuir chevelu.

Il est également décrit dans la présente invention et l'utilisation de mannoprotéines pour la préparation d'une composition dermatologique telle que définie ci-dessus ou telle qu'obtenue par le procédé de préparation ci-dessus, destinée au traitement et / ou à la prévention des conditions pathologiques de la sécheresse cutanée et/ou de l'hyperséborrhée et/ou de l'acné et / ou de la perte des cheveux.

L'utilisation telle que définie ci-dessus est destinée à une application topique de ladite composition dermatologique sur la peau et/où les phanères.

L'utilisation telle que définie ci-dessus peut consister en une ou plusieurs applications par jour, de préférence une à trois applications par jour.

La fréquence des applications de la composition dermatologique peut diminuer au cours du traitement.

Le traitement dermatologique peut consister en un traitement aigu, de quelques jours à plusieurs semaines, ou un traitement chronique sur plusieurs années. Le traitement peut également consister en un traitement sous forme de cures renouvelées tous les ans ou plusieurs fois par an.

Il est décrit dans l'invention une composition dermatologique comprenant des mannoprotéines à titre de substance active, en association avec au moins une autre substance active.

Une telle composition dermatologique est particulièrement utile dans le traitement de la sécheresse cutanée pathologique de la peau, appelée également xérose, notamment en cas d'ichtyose, de sécheresse de la peau associée à de l'eczéma ou au psoriasis ou de sécheresse pathologique du cuir chevelu, notamment associée à des pellicules.

Ladite composition dermatologique est également utile dans le traitement de l'hyperséborrhée pathologique, notamment associé à une dérégulation hormonale, notamment chez l'adolescent, la femme enceinte ou ménopausée.

Ladite composition dermatologique est également utile dans le traitement de l'acné pathologique, notamment de l'acné juvénile ou associé à une hyperséborrhée.

Ladite composition dermatologique est également utile dans le traitement de la perte pathologique des cheveux, appelée également pelade, résultant d'un choc émotif, un dysfonctionnement thyroïdien et/ou de traitements ayant comme effet secondaire d'entraîner une alopécie (par exemple les traitement anti-cancéreux).

De façon avantageuse, l'utilisation des mannoprotéines pour la préparation d'une composition dermatologique telle que définie ci-dessus n'est pas destinée au traitement de maladies impliquant la migration des neutrophiles, et notamment de maladies associées à l'IL-8.

Il est également décrit dans la présente invention l'utilisation d'une composition cosmétique ou d'une composition dermatologique telles que définies ci-dessus, destinée au traitement des effets secondaires ou aux manifestations désagréables d'autres traitements.

En particulier, lesdits effets secondaires ou manifestations désagréables se traduisent par une sécheresse de la peau, par exemple associée à de l'eczéma, une hyperséborrhée, une perte des cheveux.

Les traitements responsables desdits effets secondaires et manifestations désagréables sont par exemple destinés au traitement de maladies impliquant la migration des neutrophiles, notamment au traitement de maladies inflammatoires ou d'infections impliquant la régulation par l'IL-8 (interleukine 8), fMLP (N-formyl-methionyl-leucyl-phenylalanine), PAF (Platelet Activating Factor), C5a (glycoprotéine provenant du clivage de la partie N-terminale du complément), TNFα (Tumor Necrosis Factor α), sélectine-L.

Il est également décrit dans la présente invention une composition cosmétique ou dermatologique comprenant des mannoprotéines à titre de substance active, caractérisée en ce qu'elle ne contient pas de polyphénols.

Plus particulièrement, lorsque les mannoprotéines sont utilisées à titre de substance active, ladite substance active a un effet hydratant et/ou un effet fermeté et/ou un effet anti-vieillissement et/ou un effet anti-séborrhée et/ou un effet anti-acné et/ou un effet reconstructeur des cheveux et/ou un effet sur la brillance et/ou la douceur et/ou la croissance des cheveux.

Par l'expression « effet hydratant », on désigne une diminution de l'évaporation de la peau par un phénomène occlusif ou par une fixation de l'eau par la substance active, un effet humectant ou hygroscopique de la substance active et / ou une propriété de fixation des corps gras dans le ciment intercellulaire.

L'effet hydratant résulte en particulier au niveau de l'épiderme d'une activation de la synthèse lipidique, en particulier des phospholipides et des lipides neutres, et de la synthèse d'acide hyaluronique.

L'effet hydratant d'une composition telle que décrite ci-dessus peut être mis en évidence *in vitro* par l'étude de la synthèse lipidique et d'acide hyaluronique par les kératinocytes, comme décrit dans l'Exemple 1.

L'effet hydratant se traduit également par un effet anti-pelliculaire lors d'une application d'une composition telle que ci-dessus décrite sur le cuir chevelu.

L'effet anti-pelliculaire peut être mis en évidence par une diminution du nombre de pellicules chez un sujet traité avec une composition telle que définie ci-dessus, par exemple comme décrit dans l'Exemple 3.

Par l'expression « effet fermeté », on désigne un aspect lisse et tendu de la peau qui résulte de son soutien mécanique, notamment par les fibres de collagène et d'élastine.

La composition telle que décrite ci-dessus permet notamment d'améliorer la contraction du lattis de collagène, d'activer la synthèse d'élastine et la maturation du collagène.

Le lattis de collagène correspond à un enchevêtrement de fibres ou fibrilles de collagène.

L'effet fermeté de la composition telle que décrite ci-dessus peut être mis en évidence *in vitro* comme décrit dans l'exemple 1.

Par l'expression « effet anti-vieillissement » ou « effet anti-âge », on désigne à la fois un effet préventif pour retarder l'apparition des signes de vieillissement de la peau et un effet immédiat pour diminuer les signes de vieillissement. La composition telle que décrite ci-dessus a notamment un effet contre le vieillissement lié à l'âge et peut également avoir un effet contre le vieillissement photo-induit.

Les signes visibles du vieillissement de la peau lié à l'âge sont notamment une sécheresse cutanée, l'apparition de ridules, de rides, une diminution de l'épaisseur de la peau, ainsi qu'une perte de souplesse de la peau.

Le vieillissement de la peau lié à l'âge se traduit également par une diminution de la quantité de collagène, de leur solubilité et de leur synthèse, une diminution de la quantité d'élastine et de microfibrilles, une diminution des glycosaminoglycanes et une inactivation des fibroblastes.

L'effet anti-vieillissement de la composition telle que décrite ci-dessus résulte notamment d'une augmentation de la prolifération des fibroblastes du derme et de leur activité en terme de synthèse de collagène et de glycosaminoglycanes.

L'effet anti-vieillissement lié à l'âge peut être mis en évidence *in vitro* par l'augmentation de la synthèse de collagène et de glycosaminoglycanes par les fibroblastes du derme comme décrit dans l'exemple 1.

Les signes de vieillissement de la peau photo-induit sont notamment l'apparition de rides profondes, une peau épaisse et rêche.

Le vieillissement de la peau photo-induit se traduit notamment par une diminution de la quantité et de la solubilité du collagène, une augmentation de la quantité d'élastine et de microfibrilles, une augmentation des glycoaminoglycanes, une augmentation des cellules inflammatoires.

Il est également décrit dans la présente invention une composition cosmétique ou dermatologique telle que définie ci-dessus, dans laquelle la substance active a un effet cicatrisant, particulièrement utile pour la réparation de blessures et/ou de brûlures. L'effet cicatrisant des mannoprotéines est notamment lié à l'activation de la synthèse d'acide hyaluronique.

Par l'expression « effet anti-séborrhée », on désigne un effet de régulation de la sécrétion sébacée, de régulation de l'adsorption du sébum et / ou une action astringente permettant de resserrer les pores de la peau.

La composition telle que définie ci-dessus permet de diminuer la sécrétion de sébum.

La composition telle que définie ci-dessus permet notamment de réguler l'adsorption du sébum par adsorption lipidique.

Une telle composition est ainsi particulièrement utile dans le cadre d'une hyperséborrhée du visage et/ou une hyperséborrhée du cuir chevelu se traduisant par des cheveux dits « gras ».

La composition cosmétique telle que définie ci-dessus a un effet anti-séborrhée utile pour les peaux grasses et/ou à tendance acnéique.

Par l'expression « effet anti-acné », on désigne un effet bénéfique sur l'acné.

En particulier, l'effet bénéfique de la composition dermatologique telle que définie ci-dessus sur l'acné est lié à une régulation de la sécrétion sébacée.

Par « effet reconstructeur », on désigne l'obtention d'un aspect lisse des cheveux. La couche externe d'un cheveu, appelée cuticule, est composée d'écailles qui se recouvrent les unes les autres. Un effet reconstructeur se traduit par un relief lisse de la cuticule, alors que des cheveux abîmés ont un relief rugueux.

La composition telle que définie ci-dessus a notamment un effet gainant sur le cheveu.

L'effet reconstructeur des cheveux peut être mis en évidence par la mesure de la topographie des cheveux, comme décrit dans l'exemple 3.

Par « effet brillance », on désigne la capacité des cheveux à réfléchir la lumière et à donner aux cheveux un effet lumineux.

Par « effet douceur », on désigne la sensation de douceur des cheveux au toucher.

Par « effet sur la croissance des cheveux », on désigne une augmentation de la cinétique de croissance des cheveux.

L'effet sur la croissance des cheveux peut être mis en évidence par un test de mesure de la cinétique de pousse des cheveux, tel que décrit dans l'exemple 3.

### EXEMPLES :

### Exemple 1 : Propriétés hydratantes, anti-âge et fermeté des mannoprotéines (alternative)

### Matériel et Méthodes

### (i) Mannoprotéines

Les mannoprotéines obtenues selon le procédé décrit dans le brevet EP 1 094117 sont mises en solution dans de l'eau et sont testées à une concentration de 0,04 mg/ml, 0,08 mg/ml, 0,016 mg/ml ou 0,032 mg/ml.

### (ii) Effet hydratant

Les tests sont effectués sur des kératinocytes épidermiques humains normaux NHEK ensemencées dans les puits d'une microplaque de 96 puits dans du milieu KSFM (sans sérum). La synthèse lipidique et la synthèse d'acide hyaluronique sont évaluées en présence des différentes concentrations de mannoprotéines. Trois puits de culture sont réalisés par condition.

Du calcium est utilisé comme témoin positif de la synthèse lipidique et l'acide rétinoïque comme témoin positif de la synthèse d'acide hyaluronique.

Le témoin négatif est constitué par du milieu de culture seul.

La synthèse d'acide hyaluronique est évaluée dans le surnageant à la fin de l'incubation (72 heures à 37°C et 5 % de CO₂) par un dosage ELISA standard, selon les indications du fabricant (R&D Systems DY3614).

La synthèse lipidique est analysée par imagerie au phosphore (phosphorImaging) et la synthèse d'acide hyaluronique est évaluée par une mesure de la concentration d'acide hyaluronique libérée dans le milieu.

### (iii) Effet anti-âge

Des fibroblastes dermiques humains normaux (NHDF) et des fibroblastes dermiques humains normaux âgés (AgNHDF) sont ensemencés dans les puits d'une microplaque de 96 puits dans du milieu DMEM + 10 % de SVF. Les tests sont réalisés en milieu DMEM + 1 % de SVF. Les deux milieux sont également supplémentés de 2 mM de L-glutamine, de 50 UI/mL de pénicilline et de 50 µg/mL de streptomycine. Le SVF (sérum de veau foetal), la L-glutamine, la pénicilline et la streptomycine proviennent d'Invitrogen. La culture est effectuée à 37°C et à 5 % de CO₂. Les fibroblastes normaux sont des fibroblastes du pool F2 au 8^{ème} passage, et les fibroblastes âgés sont des fibroblastes du pool F2 au 18^{ème} passage.

Le test de prolifération des fibroblastes et le test de synthèse de glycosaminoglycane et de collagène sont effectués en présence de différentes concentrations de mannoprotéines. Trois puits de culture sont réalisés par condition.

Le témoin négatif est constitué par du milieu de culture seul.

Le test de prolifération est effectué 24h après ensemencement des cellules. De la [³H]-thymidine est ajoutée dans le milieu de culture. L'EGF est utilisé comme témoin positif

La synthèse de glycosaminoglycane et de collagène est évaluée sur des cellules à 80 % de confluence auxquelles est ajoutée respectivement de la [³H]-glucosamine ou de la de la [³H]-proline. L'acide rétinoïque est alors utilisé comme témoin positif.

Après 72h d'incubation, les macromolécules sont extraites et l'incorporation des précurseurs radioactifs est mesurée. Pour ce qui est de l'incorporation de proline, on distingue l'incorporation dans le surnageant (fraction soluble) et dans la fraction intracellulaire et déposée.

### (iv) Effet fermeté

Les tests sont effectués sur des fibroblastes dermiques humains normaux âgés (AgNHDF).

La synthèse et la maturation du collagène sont évaluées après préculture des cellules en flasque pendant 8 jours en présence de différentes concentrations de mannoprotéines. Le témoin négatif est constitué par du milieu de culture seul et le témoin positif par du TGFβ et de la vitamine C. Les cellules sont alors ensemencées en chambre de culture. Juste avant confluence, les cellules sont fixées au méthanol et la présence de collagène est détectée par immunohistochimie en utilisant un anticorps spécifique dirigé contre le collagène I et un anticorps secondaire fluorescent. Le niveau d'expression du collagène intracellulaire et extracellulaire et sa localisation autour de la matrice sont analysés au microscope.

La contraction du lattis de collagène est évaluée après culture des cellules en flasque pendant 8 jours en présence de différentes concentrations de mannoprotéines. Le témoin négatif est constitué par du milieu de culture seul et le témoin positif par du TGFβ. La suspension cellulaire obtenue est alors mise en présence d'une solution de collagène I sous pH contrôlé. Après quelques heures, la solution se gélifie de façon à obtenir un derme équivalent dont le contour est clairement défini. Le diamètre et le nombre de cellules de chaque derme équivalent sont mesurés en suivant une cinétique définie.

La synthèse d'élastine est évaluée après culture des cellules en flasque pendant 8 jours en présence de différentes concentrations de mannoprotéines. Le témoin négatif est constitué par du milieu de culture seul et le témoin positif par de la vitamine C. Les cellules sont alors ensemencées en chambres de culture. Juste avant confluence, les cellules sont fixées au méthanol et la présence d'élastine est détectée par immunohistochimie en utilisant un anticorps spécifique dirigé contre l'élastine et un anticorps secondaire fluorescent. Le niveau d'expression d'élastine est analysé au microscope.

### (v) Statistiques

Des comparaisons intergroupes sont effectuées par analyse de variance (ANOVA) à l'aide d'un test de comparaison multiple de Dunnett.

### Résultats

### i) Evaluation de l'effet hydratant sur l'épiderme

En présence de la solution de mannoprotéines, la synthèse de lipides et d'acide hyaluronique par les kératinocytes est activée par rapport au contrôle négatif.

En particulier, lorsque les kératinocytes sont soumis à une concentration de 0,032 mg/mL de mannoprotéines, on obtient une stimulation de 151 % de la production d'acide hyaluronique par rapport au témoin négatif (le test étant jugé significatif car la valeur de p est inférieure à 0,05).

### ii) Evaluation de l'effet anti-âge sur le derme

En présence de la solution de mannoprotéines, on observe une activation de la prolifération des cellules et une augmentation de la synthèse des constituants majeurs de la matrice extracellulaire (par rapport au contrôle négatif).

En particulier, on obtient les résultats présentés dans le tableau 1 ci-dessous en ce qui concerne la synthèse de collagène :

**Tableau 1 - synthèse de collagène observée en présence de mannoprotéines (MP)**

| **Conditions de test** | **Variation de la synthèse de collagène par rapport au témoin négatif** | **Valeur de p** |
|---|---|---|
| NHDF, 80 µg/mL MP, mesure sur surnageant | +7% | < 0,05 |
| NHDF, 400 µg/mL MP, mesure sur surnageant | +8% | < 0,05 |
| AgNHDF, 16 µg/mL MP, mesure sur surnageant | +12% | < 0,05 |
| AgNHDF, 80 µg/mL MP, mesure sur surnageant | +10% | < 0,05 |
| AgNHDF, 400 µg/mL MP, mesure sur surnageant | +26 % | < 0,01 |
| AgNHDF, 16 µg/mL MP, mesure sur dépôt | + 1 % | < 0,05 |
| AgNHDF, 80 µg/mL MP, mesure sur dépôt | +5 % | < 0,05 |
| AgNHDF, 400 µg/mL MP, mesure sur dépôt | +7 % | < 0,05 |

On obtient en outre les résultats présentés dans le tableau 2 ci-dessous en ce qui concerne la synthèse de glycosaminoglycane (GAG) :

**Tableau 2 - synthèse de glycosaminoglycane observée en présence de mannoprotéines (MP)**

| **Conditions de test** | **Variation de la synthèse de GAG par rapport au témoin négatif** | **Valeur de p** |
|---|---|---|
| NHDF, 80 µg/mL MP | + 6 % | < 0,05 |
| AgNHDF, 80 µg/mL MP | + 22 % | < 0,05 |
| AgNHDF, 400 µg/mL MP | + 10 % | < 0,05 |

### iii) Evaluation de l'effet fermeté sur le derme

En présence de la solution de mannoprotéines, on observe une augmentation du niveau d'expression du collagène, ainsi qu'une maturation du collagène révélée par sa déposition autour de la matrice, par rapport au témoin négatif. L'augmentation de la densité du derme équivalent (rapport diamètre sur nombre de cellules plus faible que celui du témoin négatif) traduit une meilleure contraction du lattis de collagène. De plus, la synthèse d'élastine par les fibroblastes est activée par rapport au témoin négatif. Tous ces éléments indiquent que les mannoprotéines améliorent les qualités biomécaniques du derme (notamment en terme d'élasticité et de compressibilité).

### Exemple 2 : Propriétés anti-séborrhée et anti-acné (alternative)

### Matériel et Méthodes

La solution de mannoprotéines est appliquée sur la peau ou le cuir chevelu de sujets présentant une hyperséborrhée respectivement au niveau de la peau ou du cuir chevelu.

La sécrétion de sébum est ensuite évaluée en appliquant un patch absorbant le sébum sur la partie du corps traitée. Le patch est ensuite analysé pour quantifier la sécrétion sébacée.

La sécrétion après traitement est comparée à la sécrétion chez le même sujet avant traitement.

### Résultats

La solution de mannoprotéines permet de diminuer la quantité de sébum sécrétée.

### Exemple 3 : Applications capillaires (alternative)

### Matériel et Méthodes

### (i) Mannoprotéines

Les mannoprotéines obtenues selon le procédé décrit dans le brevet EP 1 094 117 sont mises en solution dans de l'eau.

### (ii) Effet anti-pelliculaire

La solution de mannoprotéines est appliquée sur le cuir chevelu de sujets souffrant de pellicules. Après traitement avec la solution de mannoprotéines, un patch est appliqué que la zone traitée pour récupérer les pellicules du cuir chevelu.

La quantité de pellicules récupérée sur le patch est comparée avant et après traitement.

### (iii) Croissance des cheveux

La cinétique de croissance des cheveux est évaluée de la manière suivante : avant traitement, une mèche de cheveu d'un sujet est colorée depuis la racine sur 2-3 cm; la solution de mannoprotéines est alors appliquée sur le cuir chevelu ; la distance entre la racine est le début de la coloration est mesurée.

La cinétique de croissance après traitement d'un groupe de sujet traité est comparée à celle obtenue avec un groupe de sujets non traités.

### (iv) Brillance des cheveux

La brillance des cheveux est déterminée en mesurant la quantité et l'intensité de la lumière réfléchie à la surface du cheveu. A cet effet, des photographies des cheveux sont effectuées en polarisation croisée et en non polarisation. Les deux photographies sont alors converties en niveaux de gris et la brillance des cheveux est obtenue par soustraction de la lumière entre les deux photographies.

La brillance des cheveux après application de la solution de mannoprotéines est comparée à celle obtenue avant traitement.

### (v) Douceur des cheveux

La douceur des cheveux est évaluée en analyse sensorielle par un jury constitué de trois personnes qualifiées pour évaluer la douceur des cheveux au toucher.

La douceur des cheveux est notée sur une échelle de 0 à 10, la note de 0 correspondant à une absence de douceur et la note de 10 à une très grande douceur.

La douceur des cheveux après application de la solution de mannoprotéines est comparée à celle obtenue avant traitement.

### (vi) Reconstruction des cheveux

La reconstruction des cheveux est évaluée en mesurant la topographie de surface des cheveux à l'aide d'un microscope interférométrique.

Les paramètres permettant de déterminer la condition des écailles de la cuticule le long du cheveu sont les suivantes :
ouverture des écailles,
longueur des écailles et
topologie de surface, à savoir la rugosité.
La surface analysée mesure 120 x 30 µm.

La reconstruction des cheveux après application de la solution de mannoprotéines est comparée à l'état des cheveux avant traitement.

### Résultats

L'application capillaire de la solution de mannoprotéines permet d'obtenir un effet anti-pelliculaire et une augmentation de la croissance des cheveux.

La solution de mannoprotéines a également un effet réparateur du cheveu, en permettant d'améliorer la brillance, la douceur et la reconstruction du cheveu. En particulier, on observe une diminution du nombre d'ouvertures des écailles de la cuticule, une augmentation de la longueur des écailles et une diminution de la rugosité.

L' exemple 4 ci-dessous illustre l'invention sans la limiter.

### Exemple 4 : Fonction d'additif texturant et d'excipient émulsifiant (invention)

### Matériel et Méthode

### (i) Mannoprotéines

Les mannoprotéines obtenues selon le procédé décrit dans le brevet EP 1 094 117 sont mises en solution dans de l'eau.

### (ii) Caractère texturant

Le caractère texturant et les propriétés de formation de film des mannoprotéines sont évalués par différentes méthodes : analyse de la taille des particules par mesure de dispersion de la lumière laser (laserlight scattering), études de rhéologie (seuil d'écoulement, viscosité et tan d (aussi appelé DMA)), tests de stabilité (conductivité, granulométrie, viscosité, couleur et odeur) à différentes températures et durées, microscopie et étalement sur la peau.

L'effet texturant des mannoprotéines dans des formulations aqueuses est comparé à la gomme de xanthane (polysaccharide naturel) et à des carbomères.

La compatibilité des mannoprotéines avec différents systèmes d'émulsifiant est également testée. Ces systèmes émulsifiant sont :
un système anionique : ceralution H,
un système non ionique : TegoCare 450,
une lotion de filtre solaire avec du TiO2, basé sur un système non ionique, et
un gel.

Le système gel permet de tester les propriétés émulsifiantes des mannoprotéines.

### Résultats

Les texturants polymériques sont largement utilisés dans les formulations cosmétiques. Ces texturants sont des produits clé pour réaliser des formulations de type crème, gel, lotions, etc. Les texturants doivent garder leurs propriétés en présence d'une large gamme d'additifs chimiques.

L'utilisation de mannoprotéines dans des formulations avec de l'eau a été comparée à l'utilisation de produits texturants classiques (additifs hydrophiles tels que la gomme de xanthane ou les carbomères). Les mannoprotéines ont également été testées dans différents systèmes d'émulsifiant.

Les mannoprotéines ont des propriétés de texturant et sont compatibles avec les systèmes d'émulsifiants classiques. Les mannoprotéines sont également capables de former des films et fonctionnent comme des émulsionnants polymériques.

On a en outre constaté que les mannoprotéines peuvent avoir un effet texturant de manière synergique avec certains autres produits texturants, tels que la gomme de xanthane. Le tableau 3 ci-dessous fournit des résultats de mesure de viscosité d'une solution comprenant de l'eau, de la gomme de xanthane et des mannoprotéines à diverses concentrations.

**Tableau 3 - rhéologie de solutions d'eau / gomme de xanthane (GX) / mannoprotéines (MP).**

| **Composition de la solution testée** | **Viscosité en mPas** |
|---|---|
| 0 % MP +1%GX +99%eau | 4460 |
| 0,2 % MP + 0,8 % GX + 99 % eau | 8752 |
| 0,5 % MP + 0,5 % GX + 99 % eau | 4127 |
| 0 % MP + 0,5 % GX + 99,5 % eau | 2460 |
| 0,1 % MP + 0,4 % GX + 99,5 % eau | 2995 |
| 0,25 % MP + 0,25 % GX + 99,5 % eau | 1022 |

## Revendications

1. Utilisation, dans des compositions cosmétiques et/ou dermatologiques, de mannoprotéines extraites de la paroi de cellules de champignons comme agent texturant en présence de gomme de xanthane comme autre agent texturant, le rapport massique des mannoprotéines par rapport à la gomme de xanthane étant compris entre 1 : 5 et 1 : 3.

2. Utilisation selon la revendication 1, dans laquelle la composition ne contient pas de polyphénols.

3. Utilisation selon la revendication 1 ou 2, dans laquelle la composition ne contient pas d'ovomucine.

4. Utilisation selon l'une des revendications 1 à 3, dans laquelle les mannoprotéines sont issues de *Saccharomyces*, *Kluyveromyces*, *Torula* ou *Candida*, de préférence *Saccharomyces*, avantageusement *Saccharomyces cerevisiae*.

5. Utilisation selon l'une des revendications 1 à 4, dans laquelle la composition comprend de 0,01% à 20% de mannoprotéines, notamment de 0,01% à 15% de mannoprotéines, notamment de 0,01% à 10% de mannoprotéines, notamment de 0,01% à 3% de mannoprotéines.

6. Composition cosmétique ou dermatologique comprenant des mannoprotéines extraites de la paroi de cellules de champignons à titre d'agent texturant et de la gomme xanthane à titre d'autre agent texturant, ladite composition ne contenant pas de polyphénols ni d'ovomucine, le rapport massique des mannoprotéines par rapport à la gomme de xanthane étant compris entre 1:5 et 1:3.

7. Composition selon la revendication 6, **caractérisée en ce que** les mannoprotéines sont issues de *Saccharomyces*, *Kluyveromyces*, *Torula* ou *Candida*, de préférence *Saccharomyces*, avantageusement *Saccharomyces cerevisiae.*

8. Composition selon la revendication 6 ou 7, **caractérisée en ce qu'**elle comprend de 0,01 % à 20 % de mannoprotéines, notamment de 0,01 % à 15 % de mannoprotéines, notamment de 0,01 % à 10 % de mannoprotéines, notamment de 0,01 % à 3 % de mannoprotéines.

## Patentansprüche

1. Verwendung, in kosmetischen und/oder dermatologischen Zusammensetzungen, von Mannoproteinen, die aus der Zellwand von Pilzen extrahiert wurden, als Texturierungsmittel in Gegenwart von Xanthangummi als weiterem Texturierungsmittel, wobei das Gewichtsverhältnis der Mannoproteine im Verhältnis zum Xanthangummi zwischen 1:5 und 1:3 liegt.

2. Verwendung gemäß Anspruch 1, wobei die Zusammensetzung keine Polyphenole enthält.

3. Verwendung gemäß Anspruch 1 oder 2, wobei die Zusammensetzung kein Ovomucin enthält.

4. Verwendung gemäß einem der Ansprüche 1 bis 3, wobei die Mannoproteine von *Saccharomyces*, *Kluyveromyces*, *Torula* oder *Candida*, vorzugsweise *Saccharomyces*, stärker bevorzugt *Saccharomyces cerevisiae*, stammen.

5. Verwendung gemäß einem der Ansprüche 1 bis 4, wobei die Zusammensetzung 0,01% bis 20% Mannoproteine, insbesondere 0,01% bis 15% Mannoproteine, insbesondere 0,01% bis 10% Mannoproteine, insbesondere 0,01% bis 3% Mannoproteine umfasst.

6. Kosmetische oder dermatologische Zusammensetzung umfassend Mannoproteine, die aus der Zellwand von Pilzen extrahiert wurden, als Texturierungsmittel und Xanthangummi als weiteres Texturierungsmittel, wobei die Zusammensetzung weder Polyphenole noch Ovomucin enthält, und wobei das Gewichtsverhältnis der Mannoproteine im Verhältnis zum Xanthangummi zwischen 1:5 und 1:3 liegt.

7. Zusammensetzung gemäß Anspruch 6, **dadurch gekennzeichnet, dass** die Mannoproteine von *Saccharomyces*, *Kluyveromyces*, *Torula* oder *Candida*, vorzugsweise *Saccharomyces*, stärker bevorzugt *Saccharomyces cerevisiae*, stammen.

8. Zusammensetzung gemäß Anspruch 6 oder 7, **dadurch gekennzeichnet, dass** die Zusammensetzung 0,01% bis 20% Mannoproteine, insbesondere 0,01% bis 15% Mannoproteine, insbesondere 0,01% bis 10% Mannoproteine, insbesondere 0,01% bis 3% Mannoproteine umfasst.

## Claims

1. Use, in cosmetic and/or dermatological compositions, of mannoproteins extracted from the cell walls of fungi as a texturizing agent with xanthan gum as another texturizing agent, the mass ratio of the mannoproteins to the xanthan gum being comprised between 1:5 and 1:3.

2. Use according to claim 1, wherein the composition contains no polyphenols.

3. Use according to claim 1 or 2, wherein the composition contains no ovomucin.

4. Use according to one of claims 1 to 3, wherein the mannoproteins originate from *Saccharomyces*, *Kluyveromyces*, *Torula* or *Candida*, preferably *Saccharomyces,* advantageously *Saccharomyces cerevisiae*.

5. Use according to one of claims 1 to 4, wherein the composition comprises from 0.01% to 20% mannoproteins, in particular from 0.01% to 15% mannoproteins, in particular from 0.01% to 10% mannoproteins, in particular from 0.01% to 3% mannoproteins.

6. Cosmetic and/or dermatological composition comprising mannoproteins extracted from the cell walls of fungi as a texturizing agent and xanthan gum as another texturizing agent, said composition containing no polyphenols nor ovomucin, the mass ratio of the mannoproteins to the xanthan gum being comprised between 1:5 and 1:3.

7. Composition according to claim 6, **characterized in that** mannoproteins originate from *Saccharomyces*, *Kluyveromyces*, *Torula* or *Candida*, preferably *Saccharomyces,* advantageously *Saccharomyces cerevisiae*.

8. Composition according to claim 6 or 7, **characterized in that** it comprises from 0.01% to 20% mannoproteins, in particular from 0.01% to 15% mannoproteins, in particular from 0.01% to 10% mannoproteins, in particular from 0.01% to 3% mannoproteins.
